(19) Europäisches Patentamt
European Patent Office
Office européen des brevets

(11) EP 2 131 850 B1

(12) EUROPEAN PATENT SPECIFICATION

(45) Date of publication and mention
of the grant of the patent:
**29.04.2015 Bulletin 2015/18**

(51) Int Cl.:
*A61K 36/28* [(2006.01)]   *A61P 3/10* [(2006.01)]
*A23L 1/30* [(2006.01)]

(21) Application number: **08723329.2**

(22) Date of filing: **07.03.2008**

(86) International application number:
**PCT/KR2008/001292**

(87) International publication number:
**WO 2008/111763 (18.09.2008 Gazette 2008/38)**

(54) **EXTRACTS OF ASTER KORAIENSIS, AND PHARMACEUTICAL COMPOSITION AND FUNCTIONAL FOOD COMPRISING THE SAME**

EXTRAKTE VON ASTER KORAIENSIS UND DIESE ENTHALTENDE PHARMAZEUTISCHE ZUSAMMENSETZUNG UND FUNKTIONALES NAHRUNGSMITTEL

EXTRAITS DE ASTER KORAIENSIS ET COMPOSITION PHARMACEUTIQUE ET ALIMENT FONCTIONNEL LES CONTENANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.03.2007   KR 20070023690**

(43) Date of publication of application:
**16.12.2009   Bulletin 2009/51**

(73) Proprietor: **Korea Institute of Oriental Medicine Yuseoung-gu**
**Daejeon 305-811 (KR)**

(72) Inventors:
• **KIM, Jin Sook**
  **Seoul 151-050 (KR)**
• **JANG, Dae Sik**
  **Daejeon 305-810 (KR)**
• **LEE, Yun Mi**
  **Daejeon 305-810 (KR)**
• **KIM, Jong Min**
  **Daejeon 306-230 (KR)**
• **KIM, Young Sook**
  **Daejeon 305-812 (KR)**
• **SHON, Eun Jin**
  **Daejeon 305-358 (KR)**
• **KIM, Chan Sik**
  **Daejeon 305-811 (KR)**
• **KIM, Jung Hyun**
  **Seoul 150-863 (KR)**
• **KIM, Ohn Soon**
  **Daejeon 305-805 (KR)**
• **JUNG, Dong Ho**
  **Daejeon 300-090 (KR)**
• **YOO, Jeong Lim**
  **Daejeon 301-150 (KR)**
• **KIM, Joo Hwan**
  **Daejeon 305-761 (KR)**

(74) Representative: **Office Freylinger**
**P.O. Box 48**
**8001 Strassen (LU)**

(56) References cited:
**JP-A- 04 342 519**

• **PARK HEE-JUHN: "Chemistry and Pharmacological Action of Caffeoylquinic Acid Derivatives and Pharmaceutical Utilization of Chwinamul (Korean Mountainous Vegetable)", ARCHIVES OF PHARMACAL RESEARCH (SEOUL), vol. 33, no. 11, November 2010 (2010-11), pages 1703-1720, XP009147725,**
• **KIM C -S ET AL: "Slow development of diabetic cataract in streptozotocin-induced diabetic rats via inhibition of aldose reductase activity and sorbitol accumulation by use of Aster koraiensis extract", KOREAN JOURNAL OF PHARMACOGNOSY 2009 KOREAN SOCIETY OF PHARMACOGNOSY KOR, vol. 40, no. 4, December 2009 (2009-12), pages 339-344, XP009147724, ISSN: 0253-3073**

**(Cont. next page)**

- SOHN EUNJIN ET AL: "Extract of the aerial parts of Aster koraiensis reduced development of diabetic nephropathy via anti-apoptosis of podocytes in streptozotocin-induced diabetic rats.", BIOCHEMICAL AND BIOPHYSICAL RESEARCH COMMUNICATIONS 1 JAN 2010 LNKD- PUBMED:19944074, vol. 391, no. 1, 1 January 2010 (2010-01-01), pages 733-738, XP002634756, ISSN: 1090-2104
- DATABASE WPI Week 200658 Thomson Scientific, London, GB; AN 2006-565916 XP002634757, & KR 2005 048 299 A (REGIONAL OFFICE KWANGJU CHONNAM SMALL &) 24 May 2005 (2005-05-24)
- DAT N.T. ET AL.: 'Gymansterkoreayne G, a new inhibitory polyacetylene against NFAT transcription factor from Gymanster koraiensis' CHEM. PHARM. BULL. vol. 53, no. 9, 2005, pages 1194 - 1196, XP008117800
- JUNG H.J. ET AL.: 'Gymansterkoreaynes A-F, cytotoxic polyacetylenes from Gymnaster koraiensis' J. NAT. PROD. vol. 65, no. 6, 2002, pages 897 - 901, XP008117799

**Description**

**Technical Field**

The present invention relates to a leaf and stem extract of *Aster koraiensis,* a root extract of *Aster koraiensis,* a flower extract of *Aster koraiensis,* and a pharmaceutical composition and functional food for use in the prevention or treatment of diabetic complications, comprising at least one of said extracts as an active ingredient, and more particularly to a leaf and stem extract of *Aster koraiensis,* a root extract of *Aster koraiensis* and a flower extract of *Aster koraiensis,* which have the effects of not only inhibiting the production of advanced glycation endproducts, which are important factors inducing diabetic complications, but also inhibiting aldose reductase activity, and thus are effective for use in the prevention and treatment of diabetic complications, and to a pharmaceutical composition and functional food for use in the prevention or treatment of diabetic complications.

**Background Art**

Diabetes is one of the important adult diseases in the world, and recently, its prevalence rate in Korea reaches 7 to 8% along with the rapid economical growth. Particularly, since the general life span is extended until diabetes is induced, diabetics cannot avoid the pain of diabetic complications. Generally, in 10 to 20 years after the onset of diabetes attack, most of the organs in the body are damaged, leading to diabetic retinopathy, diabetic cataract, diabetic nephropathy, diabetic neuropathy and the like. Chronic diabetic nephropathy is a critical cause of hemodialysis and end-stage renal failure, and diabetic cataract causes loss of eyesight, leading to death.

Mechanisms inducing such diabetic complications are generally explained by non-enzymatic glycation of protein, polyol pathway and oxidative stress.

The nonenzyme glycation of protein is caused by the nonenzymatic condensation of the amino acid groups (e.g., lysine residues) of protein with reducing sugar without enzymatic action, that is, Millard reaction. As a result of the reaction, advanced glycation endproducts (AGEs) are produced.

The nonenzymatic glycation of protein can consist of two steps. In the first step, the amino acid groups (e.g., lysine residues) of protein, and the aldehydes or ketones of reducing sugar, are subjected to a nucleophilic addition reaction without the action cf enzymes so as to form Schiff bases as early-stage products, and ketoamine adducts adjacent to the Schiff bases are condensed with each other to produce reversible Amadori-type early glycation products. In the second step, by the continuation of a high blood glucose condition, the reversible Amadori-type early glycation products are rearranged without degradation and cross-linked with proteins, thus producing advanced glycation endproducts.

Unlike the reversible Amadori-type early glycation products, AGEs are irreversible products. Thus, even when blood glucose is recovered to a normal level, the endproducts, once produced, are accumulated in tissues during a protein existing period without degradation, resulting in an abnormal change in the structure and function cf the tissues (Vinson, J. A. et al., 1996, J. Nutritinal Biochemistry 7: 559-663; Smith, P. R. et al., 1992, Eur. J. Biochem., 210: 729-739).

For example, glycated albumin, which is one cf advanced glycation endproducts produced by the reaction of glucose with various proteins, acts as an important factor causing chronic diabetic nephropathy. The glycated albumin is introduced into glomerular cells more easily than non-glycated normal albumin, and a high concentration cf glucose stimulates glomerular cells to increase the synthesis of extracellular matrices. The excessive introduction cf glycated albumins and the increase cf extracellular matrices result in the fibrillation cf glomerules. By such mechanisms, the glomerules are continuously damaged, eventually making the use cf an extreme treatment method, and thus extreme treatment methods such as blood dialysis or organ transplantation are unavoidable. Also, it was reported that, due to chronic diabetes, collagen in the arteries wall and basement membrane protein in the renal glomerulus bind to AGEs to be accumulated in the tissue (Brownlee, M., et al., 1986, Sciences, 232, 1629-1632).

As described above, the nonenzymatic protein glycation leads to the glycation of basement membranes, serum albumins, lens proteins, fibrins, collagens, etc., and the advanced glycation endproducts cause an abnormal change in the structure and function of the tissues, resulting in chronic diabetic complications, such as diabetic retinopathy, diabetic cataract, diabetic nephropathy, diabetic neuropathy, etc.

Also, it was reported that, during the production of AGEs in the high blood sugar state, dyslipidemia occurs and, at the same time, the function of a defense system against harmful oxygen free radicals is deteriorated, thus inducing oxidative stress (Yokozawa, T., et al, 2001, J. of Trad. Med., 18: 107-112). Thus, the non-enzymatic glycation reaction is associated with the action mechanism cf oxidative stress.

The polyol pathway includes two steps. In the first step, sorbitol is formed from aldose or ketose by the action cf aldose reductase (AR). In the second step, sorbitol is oxidized by sorbitol dehydrogenase to produce fructose. The polyol pathway is activated by a high blood glucose level, whereby sorbitol and fructose are excessively produced and accumulated in tissue, thus increasing the osmotic pressure cf the tissue. Due to the increased osmotic pressure, water is introduced into the tissue to cause diabetic retinopathy, diabetic cataract, diabetic neuropathy and the like (diabetics, Eungjn Kim et al., Korean diabetes association, Korea medical, 483p; Soulis-Liparota, T., et al., 1995, Di- abetologia, 38: 357-394).

It was reported that AGEs activate aldose reductase (AR), which is a key enzyme cf the polyol pathway in human microvascular endothelial cells (Nakamura, N., et al., 2000, Free Radic Biol. Med., 29: 17-25). In the normal state, AR shows a low affinity for glucose, but at high glucose concentrations, AR is activated to excessively reduce glucose to sorbitol, which is then converted into fructose by sorbitol dehydrogenase. Fructose shows non-enzymatic glycation rate which is about 10-fold higher than that cf glucose. Therefore, a high concentration cf fructose binds to protein, thus accelerating the formation cf AGEs.

As described above, diabetic complications are induced by interaction between the mechanisms of non-enzymatic glycation, polyol pathway and oxidative stress. Therefore, in order to prevent or treat diabetic complications, or delay the onset thereof, it is very important to inhibit the formation cf advanced glycation end products and, at the same time, inhibit aldose reductase activity (Brownlee, M., et al., 1988, N. Engl. Med, 318, 1315-1321).

When a blood glucose level is not immediately lowered due to the onset of diabetes, a drug for preventing diabetic complications must be administered to prevent diabetic complications.

Aaminoguanidine, which is the only synthetic drug currently used as a protein glycosylation inhibitor, is a nucleophilic hydrazine. It prevents Amadori products from crosslinking with proteins, by binding to the Amadori products, so as to inhibit the production cf advanced glycation products, thus delaying or preventing the development cf diabetic complications (Brownlee, M., et al., 1986, Sciences, 232, 1629-1632; Edelstein, D. et al., 1992, Diabetes, 41, 26-29). The aminoguanidine, which is the most promising synthetic drug candidate for the prevention and treatment of diabetic complication, was developed up to third-phase clinical trials but has the problem of causing toxicity upon long-term administration. Thus, the development of safer drugs is now needed.

Park Hee-Juhn describes in "Chemistry and Pharmacological Action of Caffeoylquinic Acid Derivatives and Pharmaceutical Utilization of Chwinamul (Korean Mountainous Vegetable)", Archives Of Pharmacol. Research (Seoul), vol. 33, no. 11, November 2010 (2010-11), pages 1703-1720, and thus published after the filing date of the present patent application, the use of Chwinamul as a side dish or a medicinal herb to treat a number of diseases, among which diabetes is explicitly cited. Chwinamul consists of the leaves of various species of wild flowering plants, but its composition may vary significantly depending on the season and the region. This documents cites a list of vegetables that may be included in Chwinamul, among which *Aster koraiensis.*

Accordingly, the present inventors have found that a leaf and stem extract of *Aster koraiensis,* a root extract of *Aster koraiensis* and a flower extract of *Aster koraiensis* inhibits the production of advanced glycation endproducts and the activity of aldose reductase, and thus can be used in the treatment of prevention of diabetic complications, thereby completing the present invention.

## Disclosure of Invention

### Technical Problem

It is an object of the present invention to provide an extract of *Aster koraiensis* for use in the treatment or the prevention of diabetic complications, preferably a leaf and stem extract of *Aster koraiensis,* a root extract of *Aster koraiensis* and a flower extract of *Aster koraiensis,* which have the effects of inhibiting the production of advanced glycation endproducts, the causes of diabetic complications, and inhibiting the activity of aldose reductase.

It is another object of the present invention to provide fractions obtained by fractionating each of said extracts in the order of hexane, ethyl acetate, n-butanol and water fractions.

It is still another object of the present invention to provide a pharmaceutical composition for use in the prevention or treatment of diabetic complications, a health functional food for use in the improvement of diabetic complications, and a non-therapeutic use for the prevention or delay of aging, each of said compositions and foods comprising, as an active ingredient, at least one of said extracts and fractions.

### Technical Solution

According to the present invention, each of extracts, may be obtained by finely dividing each of the leaf and stem part, root and flower of *Aster koraiensis,* extracting the cut plant with alcohol or aqueous alcohol solution, filtering the extracted solution and concentrating the filtrate under reduced pressure, can be used in a pharmaceutical composition for use in the prevention or treatment of diabetic complications, a non-therapeutic use for the prevention of aging, or a health functional food for use in the improvement of diabetic complications.

Also, each of fractions, obtained by fractionating each of said leaf and stem extract, root extract and flower extract in the order of hexane, ethyl acetate, n-butanol and water fractions, can be used in a pharmaceutical composition of diabetic complications, or a health functional food for use in the improvement of diabetic complications.

**Advantageous Effects**

As described above, the leaf and stem extract of *Aster koraiensis,* the root extract of *Aster koraiensis,* the flower extract of *Aster koraiensis,* and the ethyl acetate fraction, butanol fraction and water fraction of each of the extracts, inhibited the production of advanced glycation endproducts, the cause of diabetic complications, and, at the same time, inhibited the activity of aldose reductase, and the inhibitory activity of said extracts and fractions were excellent compared to those of positive control groups, aminoguanidine and 3,3-tetramethyleneglutaric acid.

Accordingly, the leaf and stem extract of *Aster koraiensis,* the root extract of *Aster koraiensis,* the flower extract of *Aster koraiensis,* and the ethyl acetate fraction, butanol fraction and water fraction of each of the extracts, can be applied in a pharmaceutical composition and functional food for use in the prevention or treatment of diabetic complications that are caused by the production of advanced glycation endproducts and the activation of aldose reductase, including diabetic retinopathy, diabetic cataract, diabetic nephropathy and diabetic neuropathy. Also, if the production of advanced glycation endproducts and the activity of aldose reductase are inhibited, the rate of induction of oxidative stress will be reduced. Thus, said extracts and fractions can be applied in a pharmaceutical composition and functional food for use in the prevention or delay of aging resulting from oxidative stress.

**Brief Description of the Drawings**

FIG. 1 is a schematic diagram showing the extraction and fractionation of *Aster koraiensis.*

FIG. 2 is a graphic diagram showing the inhibitory effect of the leaf and stem extract of *Aster koraiensis* on the production of advanced glycation endproducts.

FIG. 3 is a graphic diagram showing the inhibitory effect of the root extract of *Aster koraiensis* on the production of advanced glycation endproducts.

FIG. 4 is a graphic diagram showing the inhibitory effect of the flower extract of *Aster koraiensis* on the production of advanced glycation endproducts.

FIG. 5 is a graphic diagram showing the inhibitory effect of the flower extract of aminoguanidine on the production cf advanced glycation endproducts.

FIG. 6 is a graphic diagram showing the inhibitory effect of the leaf and stem extract cf *Aster koraiensis* on the activity cf aldose reductase.

FIG. 7 is a graphic diagram showing the inhibitory effect cf the root extract cf *Aster koraiensis* on the activity cf aldose reductase.

FIG. 8 is a graphic diagram showing the inhibitory effect cf the flower extract cf *Aster koraiensis* on the activity of aldose reductase.

FIG. 9 is a graphic diagram showing the inhibitory effect cf 3,3-tetramethyleneglutaric acid on the activity cf aldose reductase.

FIG. 10 is a graphic diagram showing the inhibitory effect cf an ethyl acetate fraction of the leaf and stem extract of *Aster koraiensis* on the activity of aldose reductase.

FIG. 11 is a graphic diagram showing the inhibitory effect of a butanol fraction cf the leaf and stem extract of *Aster koraiensis* on the activity of aldose reductase.

FIG. 12 is a graphic diagram showing the inhibitory effect cf a water fraction cf the leaf and stem extract cf *Aster koraiensis* on the activity cf aldose reductase.

FIG. 13 is a graphic diagram showing the inhibitory effect of an ethyl acetate fraction of the root extract cf *Aster koraiensis* on the activity cf aldose reductase.

FIG. 14 is a graphic diagram showing the inhibitory effect of a butanol fraction cf the root extract cf *Aster koraiensis* on the activity cf aldose reductase.

FIG. 15 is a graphic diagram showing the inhibitory effect of an ethyl acetate fraction cf the flower extract of *Aster koraiensis* on the activity of aldose reductase.

FIG. 16 is a graphic diagram showing the inhibitory effect of a butanol fraction of the flower extract cf *Aster koraiensis* on the activity of aldose reductase.

FIG. 17 is a graphic diagram showing the AGEs-BSA production-inhibitory effect of the root extract cf *Aster koraiensis,* measured using the ELISA assay.

FIG. 18 is a graphic diagram showing the AGEs-BSA production-inhibitory effect of the stem and leaf extract of *Aster koraiensis,* measured using the ELISA assay.

FIG. 19 is a graphic diagram showing the AGEs-BSA production-inhibitory effect of the flower extract cf *Aster*

*koraiensis,* measured using the ELISA assay.

**Mode for the Invention**

To achieve the above objects, the present invention provides a leaf and stem extract cf *Aster koraiensis,* a root extract cf *Aster koraiensis* and a flower extract of *Aster koraiensis,* which are obtained by drying and finely cutting each cf the leaf and stem part, root and flower of *Aster koraiensis,* extracting the cut plant with alcohol or aqueous alcohol solution, filtering the extracted solution, and concentrating the filtrate.

In another aspect, the present invention provides an ethyl acetate fraction, butanol fraction and water fraction of each of said *Aster koraiensis* extracts, obtained by fractionating each of the extracts.

In still another aspect, the present invention provides a pharmaceutical composition for use in the prevention or treatment of diabetic complications, a health functional food for use in the improvement of diabetic complications, and a non-therapeutical use of an extract of *Aster koraiensis* for the prevention or delay of aging, each of said compositions and foods containing, as active ingredient, at least one of said extracts and fractions.

*Aster koraiensis* is a perennial plant belonging to the family Compositae, is 50-60 cm in height, and generally grows in relatively wet land. It is also planted in gardens (Sei-Young, Yun, Pictorial Book of Korean Plants, Academic Press, pp. 508). However, a report of the effect of *Aster koraiensis* on the prevention or treatment of diabetic complications, or a report of any effects or components thereof, has not yet been reported.

Accordingly, the present inventors have found that the leaf and stem extract of *Aster koraiensis,* the root extract of *Aster koraiensis,* the flower extract of *Aster koraiensis,* and the ethyl acetate fraction, butanol fraction and water fraction of each of said *Aster koraiensis* extracts, showed excellent inhibitory effects on the production of diabetic complications, thereby completing the present invention.

Hereinafter, the present invention will be described in detail.

According to the present invention, the leaf and stem extract of *Aster koraiensis,* the root extract of *Aster koraiensis* and the flower extract of *Aster koraiensis* are prepared by drying and finely cutting each of the leaf and stem part, root and flower of *Aster koraiensis,* extracting the cut plant with alcohol or aqueous alcohol solution, filtering the extracted solution and concentrating the filtrate under reduced pressure.

The method for preparing the *Aster koraiensis* extracts and the method for examining the effects of the extracts will be described in further detail.

1. Method for preparing *Aster koraiensis* extracts

1) Extraction step

First, the leaf and stem of *Aster koraiensis* are cut into fine pieces. To the cut leaf and stem, a 5-104>ld amount (w/v) of any one selected from among methyl alcohol, ethyl alcohol, and aqueous solutions thereof, is added. The plant-containing solution is continuously extracted 2-5 times at room temperature.

2) Concentration step

The extracted solution is filtered, and then concentrated under reduced pressure. At this time, the concentration temperature is maintained at 40-45 °C, such that the decomposition and hydrolysis cf the plant can be prevented.

The root of *Aster koraiensis* and the flower cf *Aster koraiensis* are also extracted in the same manner as described above.

2. Step cf fractionating extract cf *Aster koraiensis*

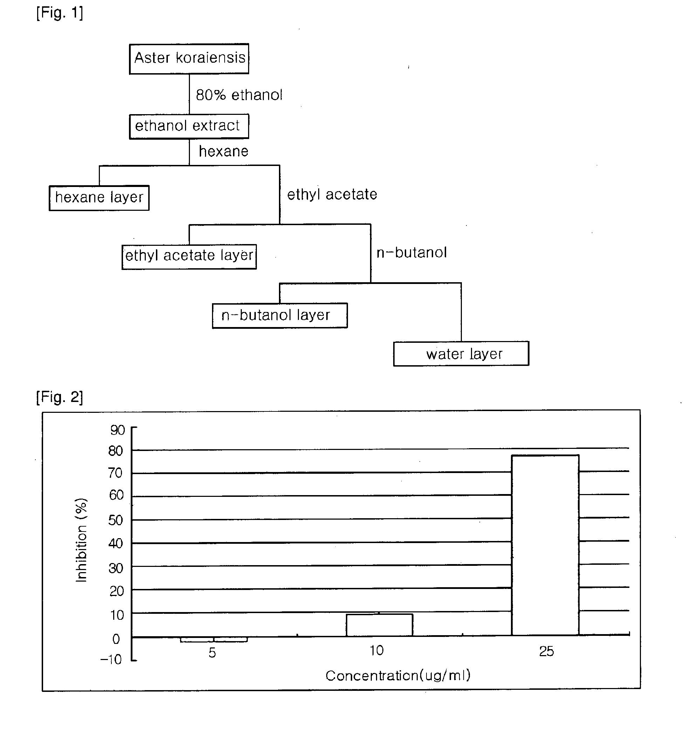

As shown in FIG. 1, each of the leaf and stem extract of *Aster koraiensis,* the root extract cf *Aster koraiensis* and the flower extract of *Aster koraiensis,* prepared as described above, is fractionated into a n-hexane layer, an ethyl acetate layer, n-butanol layer and a water layer, thus obtaining a n-hexane fraction, ethyl acetate fraction, n-butanol fraction and water fraction of each of the extracts.

3. Effect of inhibiting advanced glycation endproducts

The leaf and stem extract cf *Aster koraiensis,* the root extract cf *Aster koraiensis,* the flower extract of *Aster koraiensis,* and the hexane fraction, ethyl acetate fraction, butanol fraction and water fraction cf each cf the extracts, were tested *in vitro.* As a result, it was observed that the extracts and the fractions inhibited the glycation cf bovine serum albumin (BSA).

Specifically, when each of the extracts and the fractions was cultured in BSA, the concentrations of each of the extracts and the fractions, at which the production of advanced glycation endproducts was inhibited by 50% ($IC_{50}$), were 18.74 $\mu$g/m$\ell$ (leaf and stem extract), 38.75 $\mu$g/m$\ell$ (root extract) and 35.36 $\mu$g/m$\ell$ (flower extract), indicating that the extracts had excellent inhibitory effects on the production of advanced glycation endproducts.

4. Effect cf inhibiting aldose reductase activity

The leaf and stem extract cf *Aster koraiensis,* the root extract of *Aster koraiensis,* the flower extract of *Aster koraiensis,* and the hexane fraction, ethyl acetate fraction, butanol fraction and water fraction cf each of the extracts, were tested *in vitro* in order to examine the effect cf inhibiting the activity of aldose reductase. As a result, it was observed that the extracts and the fractions inhibited the activity cf aldose reductase.

Specifically, the concentrations of each of the extracts and the fractions, at which the activity cf aldose reductase was inhibited by 50%, were 0.40 $\mu$g/m$\ell$ (leaf and stem extract), 3.77 $\mu$g/m$\ell$ (root extract) and 2.07 $\mu$g/m$\ell$ (flower extract), indicating that the extracts had excellent inhibitory effects on the activity of aldose reductase.

Also, the 50% inhibitory concentrations of the fractions of the leaf and stem extract for aldose reductase activity were 1.34 $\mu$g/m$\ell$ (ethyl acetate fraction), 0.47 $\mu$g/m$\ell$ (butanol fraction) and 2.15 $\mu$g/ml, (water fraction).

Furthermore, the 50% inhibitory concentrations of the fractions of the root extract for aldose reductase activity were 0.41 $\mu$g/ml (ethyl acetate fraction) and 0.86 $\mu$g/ml (butanol fraction).

In addition, the 50% inhibitory concentrations of the fractions of the flower extract for aldose reductase activity were 1.39 $\mu$g/ml, (ethyl acetate fraction) and 3.63 $\mu$g/ml, (butanol extract).

In another aspect, the present invention provides a pharmaceutical composition for use in the prevention or treatment of diabetic complications, and a pharmaceutical composition for prevention or delay of aging, each of the compositions containing, as an active ingredient, at least one selected from among the leaf and stem extract of *Aster koraiensis,* the root extract of *Aster koraiensis,* the flower extract of *Aster koraiensis,* the ethyl acetate fraction, butanol fraction and water fraction of each of the extracts. The pharmaceutical compositions may be administered orally in clinical trials and used in the form of general drug formulations.

Specifically, the pharmaceutical compositions according to the present invention may be administered in various formulations, which are prepared using diluents or excipients such as filler, extender, bonding agent, wetting agent, disintegrating agent, surfactant, etc. Solid formulations for oral administration include tablets, pills, powders, granules, capsules, etc. and are prepared by mixing one or more excipients, for example, starch, calcium carbonate, sucrose or lactose, gelatin, etc., with the above extract or fraction.

Also, besides simple excipients, lubricant such as magnesium stearate or talc may be used. Solutions for oral administration include suspension, internal solution, emulsion, syrup, etc., and besides simple diluents such as water and liquid paraffin, various excipients, for example, wetting agents, sweetening agents, odorant, preservatives, etc. may be used.

Formulations for parenteral administration include sterilized aqueous solutions, nonaqueous solutions, suspensions, emulsions, lyophilized preparations, or suppositories. As non-aqueous solutions and suspensions, propylene glycol, polyethylene glycol, vegetable oil such as olive oil, injectable ester such as ethylolate, etc. may be used. As suppository bases, Witepsol, macrogol, tween 61, cacao butter, laurin butter, glycero gelatin, etc. may be used.

In still another aspect, the present invention provides a functional food for use in the prevention or treatment of diabetic complications, and a non-therapeutic use for the prevention or delay of aging, each of the compositions containing, as an active ingredient, at least one selected from among the leaf and stem extract of *Aster koraiensis ,* the root extract of *Aster koraiensis,* the flower extract of *Aster koraiensis,* the ethyl acetate fraction, butanol fraction and water fraction of each of the extracts. The functional foods according to the present invention may contain food additives depending on the intended use of final products and the preference of consumers,

It was reported that, during the production of advanced glycation endproducts, abnormality in lipid metabolism occurs and, at the same time, the function of a defense system against harmful oxygen free radicals is deteriorated, thus inducing oxidative stress. Meanwhile, due to oxidative stress, oxygen species, which have been present in a stable form in vivo, are converted into highly reactive active oxygen species by environmental and biochemical factors, including enzyme systems, reductive metabolisms, chemicals, pollutants and photochemical reactions, thus irreversibly breaking the proteins, lipids and DNA of human cells. It is considered that, when the production of advanced glycation endproducts is inhibited, the rate of induction of oxidative stress will be reduced.

Example: Extraction and fractionation of *Aster koraiensis*

On September, 2004, the leaf, stem, root and flower of *Aster koraiensis* were collected in Muju-gun, Jeollabuk-do, Korea. The collected plant was identified and divided into the leaf and stem part, the root and flower, each of which was then cut into fine pieces. Then, each of the cut plant parts was added to 80% ethanol and extracted three times in an extractor at room temperature for 7 days each time. Each of the extracts was concentrated under reduced pressure, thus obtaining a leaf-stem extract, a root extract and a flower extract. In the concentration step, the water bath in the extractor was maintained at 40-45 $^0$C in order to prevent the decomposition and hydrolysis of the components of the plant. After the ethyl alcohol was completely removed through the vacuum concentration, the extracts were dried in a freeze dryer to remove water.

As shown in FIG. 1, the leaf and stem extract, the root extract and the flower extract, obtained as described above, were fractionated into n-hexane, ethyl acetate, n-butanol and water layers, and then the feaction of each of the layers was collected.

Test Example 1: Analysis cf inhibitory effect of inventive materials on advanced glycation endproducts

For the *in vitro* analysis of the inhibitory effect cf each cf the leaf and stem extract of *Aster koraiensis,* the root extract of *Aster koraiensis,* the flower extract of *Aster koraiensis,* and the fractions cf each of the extracts, on the production cf advanced glycation endproducts, bovine serum albumin (hereinafter, referred to as BSA; Sigma, USA)) was selected as a protein source. BSA was added to 50 mM phosphate buffer (pH 7.4) to a concentration of 10 mg/m$\ell$. As a sugar source, a mixture of 0.2 M fructose and 0.2 M glucose was used. The fructose/glucose mixture was added to the prepared BSA solution. As test groups, each of the leaf and stem extract cf *Aster koraiensis,* the root extract cf *Aster koraiensis,* the flower extract of *Aster koraiensis,* and the hexane fraction, ethyl acetate fraction, butanol fraction and water fraction of each cf the extracts, was dissolved in distilled water. Then, each of the solutions was added to the BSA/sugar mixture and cultured at 37 °C for 14 days.

At this time, 0.02% sodium azide was added as an antibacterial agent. A control group was a culture of the BSA/sugar mixture, and the blank of each cf the test groups and the control group was not cultured after their preparation. Meanwhile, as a positive control for use as an index to determine the superiority cf the inventive material, aminoguanidine was used. Four samples for each of the cultures were prepared to minimize errors. After 14 days, the content of advanced glycation endproducts (AGEs) in each of the cultures was analyzed, and the analysis results were recorded. AGEs show a fluorescent brown color and have the physical and chemical properties capable of cross-linking. Also, they have ligands which can be recognized by cell membrane receptors. The amount cf advanced glycation endproducts having such properties was measured with a spectrofluorometer (excitation: 350 nm, and emission: 450 nm) to analyze the inhibition of the production of advanced glycation endproducts.

The leaf and stem extract of *Aster koraiensis,* prepared in Example above, was prepared into solutions having concentrations of 5 $\mu$g/m$\ell$, 10 $\mu$g/m$\ell$ and 25 $\mu$g/m$\ell$, respectively. Then, each cf the prepared solutions was cultured at 37 °C for 14 days according to the method described in Test Example 1.

Each of the root extract and flower extract of *Aster koraiensis,* prepared in Example, were prepared into solutions having concentrations cf 10 $\mu$g/m$\ell$, 25 $\mu$g/m$\ell$ and 50 $\mu$g/m$\ell$, respectively. Then, each of the prepared solutions was cultured at 37 °C for 14 days according to the method described in Test Example 1. After 14 days, the amount of advanced glycation endproducts produced in each of the cultures was measured with a spectrcfluorometer (excitation: 350 nm, and emission: 450 nm), and the measurement results are shown in Table 1 and FIG. 2.

The inhibition of the production of advanced glycation endproducts is calculated according to the following equation:

$$\text{Inhibition (\%) cf production} = 100 - \text{(fluorescent intensity cf sample group}$$
$$\text{fluorescent intensity cf blank cf sample group)/(fluorescent intensity cf control group} -$$
$$\text{fluorescent intensity cf blank cf control group)x100}$$

Comparative Example 1: Inhibitory effect of aminoguanidine on production of advanced glycation endproducts

As a positive control group, aminiguanidine, which is the most promising synthetic drug for the prevention and treatment cf diabetic complications, was dissolved in distilled water to concentrations of 18.5 $\mu$g/m$\ell$, 55.5 $\mu$g/m$\ell$ and 74.0 $\mu$g/m$\ell$, and then cultured at 37 °C for 14 days according to the method described in Test Example 1.

After 14 days, the amount cf advanced glycation endproducts produced in each cf the cultures was measured with a spectrcfluorometer (excitation: 350 nm, and emission: 450 nm).

Table 1

[Table 1]

| Samples | Inhibition (%) of production of advanced glycation endproducts | | IC$_{50}$ inhibitory concentration ($\mu$g/ml) | See |
|---|---|---|---|---|
| | Concentration | Inhibition | | |
| Leaf and stem extract of *Aster koraiensis* | 5 | -1.83 ± 1.37 | 18.74 | FIG. 2 |
| | 10 | 9.44 ± 0.89 | | |
| | 25 | 76.66 ± 0.46 | | |
| Root extract of *Aster koraiensis* | 10 | -3.90 ± 0.57 | 38.75 | FIG. 3 |
| | 25 | 15.01 ± 0.45 | | |
| | 50 | 75.47 ± 0.82 | | |
| Flower extract of *Aster koraiensis* | 10 | 2.33 ± 1.20 | 35.36 | FIG. 4 |
| | 25 | 32.51 ± 0.63 | | |
| | 50 | 76.36 ± 0.45 | | |
| Aminoguanidine | 18.5 | 12.70 ± 6.12 | 72.12 | FIG. 5 |
| | 55.5 | 39.46 ± 1.91 | | |
| | 74.0 | 50.68 ± 0.51 | | |

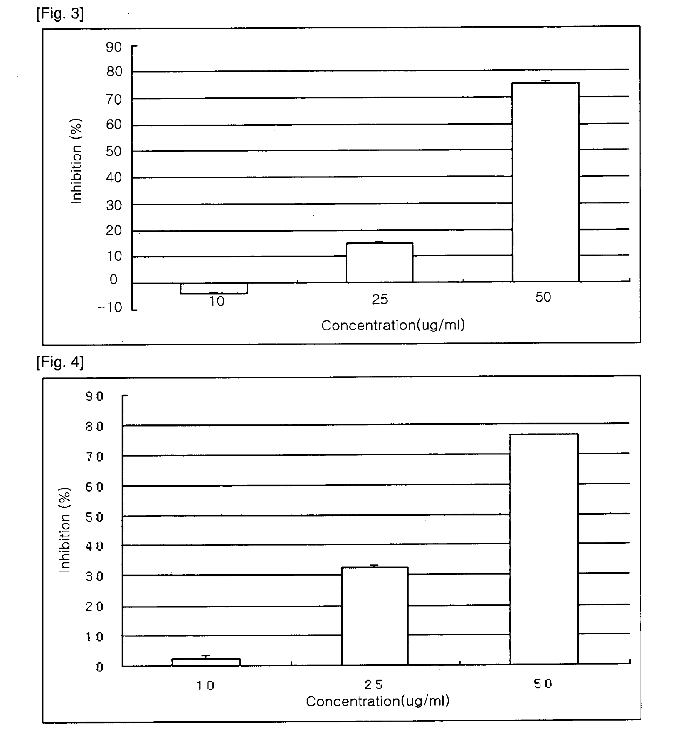

As shown in Table 1 and FIGS. 2 to 5, the leaf and stem extract of *Aster koraiensis* showed an IC$_{50}$ value of 18.74 $\mu$g/m$\ell$, and thus the inhibitory effect thereof was about 3.8-fold superior to the positive control aminoguanidine (IC$_{50}$ of 72.12 $\mu$g/m$\ell$). Also, the effects of the root extract (IC$_{50}$ of 38.75 $\mu$g/m$\ell$) and the flower extract (IC$_{50}$ of 35.36 $\mu$g/m$\ell$) were about 2-fold superior to the positive control aminoguanidine.

Test Example 2: Analysis of inhibitory effect of inventive extracts and fractions on aldose reductase activity

The leaf and stem extract cf *Aster koraiensis*, the root extract of *Aster koraiensis,* the flower extract of *Aster koraiensis,* and the hexane fraction, ethyl acetate fraction, butanol fraction and water fraction of each of the extracts, were analyzed *in vitro* for their inhibitory effects on aldose reductase activity.

[Test method]

According to the method of Dufrane (1984), in order to obtain natural aldose reductase from the eyeballs of SD rats (Sprague-Dawley rats, 250-280 g), the lenses collected from the rats were crushed in 135 mM Na, K-phosphate buffer (pH 7.0) and 10 mM 2-mercaptoethanol using a homogenizer and a sonicater. The resulting solution was centrifuged at 14000 rpm for 30 minutes, and the supernatant was used in the experiment, after it was filtered through a 0.2-$\mu$m filter. The above procedures were all carried out at 4 °C. BSA was used as an enzyme protein source, and the Lawry's method was used to determine protein concentrations. A mixture of 135 mM Na, K-phosphate buffer (pH 7.0), 100 mM lithium sulfate, 0.03 mM NADPH, 0.04 mM DL-glyceraldehyde and 100 $\mu$g/m$\ell$ enzyme was dissolved in 0.1% DMSO, and then added to 50 $\mu\ell$ of each of the samples to make a total volume cf 1 m$\ell$. Then, the resulting solution was allowed to react at 37 °C for 10 minutes. As a blank, a mixture, to which 0.04 mM DL-glycealdehyde has not been added, was used, and as a standard, a mixture cf 135 mM Na, K-phosphate buffer (pH 7.0) and 100 mM lithium sulfate, to which 50 $\mu\ell$ of NADP (0.2-5 $\mu$m) has been added, was used. After 0.3 m$\ell$ of 0.5 N HCl was added to the sample to stop the reaction, 1 m$\ell$ of 6 M NaOH containing 10 mM imidazole was added thereto and allowed to react at 60 °C for 10 minutes. The conversion of NADP to fluorescent products was measured. The measurement cf each sample was carried out in triplicate. The effect of each sample was measured with a spectrcfluorophotometric detector (Bio-TEK, Synergy HT, USA) at an excitation wavelength cf 360 nm and an emission wavelength of 460 nm, and was expressed as IC$_{50}$

1. Analysis of aldose reductase activity-inhibitory effect of leaf and stem extract of *Aster koraiensis,* root extract cf *Aster koraiensis* and flower extract of *Aster koraiensis*

The leaf and stem extract of *Aster koraiensis,* prepared in Example, was prepared into solutions having concentrations cf 0.1 $\mu$g/m$\ell$, 0.25 $\mu$g/m$\ell$ and 0.5 $\mu$g/m$\ell$, respectively. Then, each the solutions was tested according to the method cf Test Example 2 to measure the effect thereof.

The root extract cf *Aster koraiensis,* prepared in Example, was prepared into solutions having concentrations of 0.5 µg/mℓ, 5 µg/mℓ and 10 µg/mℓ, respectively. Then, each cf the solutions was tested according to the method cf Test Example 2 to measure the effect thereof.

The flower extract cf *Aster koraiensis,* prepared in Example, was prepared into solutions having concentrations of 1.0 µg/mℓ, 2.5 µg/mℓ and 5 µg/mℓ, respectively. Then, each of the solutions was tested according to the method of Test Example 2 to measure the effect thereof.

Comparative Example 2: Aldose reductase activity-inhibitory activity of 3,3-tetramethyleneglutaric acid

As a comparative group, 3,3-tetramethyleneglutaric acid, which is an excellent inhibitor of aldose reductase, was measured for its aldose reductase activity-inhibitory effect according to the method cf Test Example 1.

Table 2

[Table 2]

| Samples | Inhibition (%) of production of advanced glycation endproducts | | $IC_{50}$ inhibitory concentration (µg/ml) | See |
|---|---|---|---|---|
| | Concentration | Inhibition | | |
| Leaf and stem extract of *Aster koraiensis* | 0.1 | 0 | 0.40 | FIG. 6 |
| | 0.25 | 25.34 $\pm$ 8.72 | | |
| | 0.5 | 67.81 $\pm$ 8.72 | | |
| Root extract of *Aster koraiensis* | 0.5 | 19.00 + 5.66 | 3.77 | FIG. 7 |
| | 5 | 75.50 $\pm$ 7.78 | | |
| | 10 | 83.50 $\pm$ 0.71 | | |
| Flower extract of *Aster koraiensis* | 1.0 | 39.76 $\pm$ 2.78 | 2.07 | FIG. 8 |
| | 2.5 | 55.12 $\pm$ 3.34 | | |
| | 5.0 | 75.20 $\pm$ 5.01 | | |
| 3,3-tetramethyleneglutaric acid | 3.72 | 39.34 $\pm$ 4.17 | 4.50 | FIG. 9 |
| | 4.66 | 55.74 $\pm$ 3.25 | | |
| | 5.59 | 60.33 $\pm$ 1.39 | | |

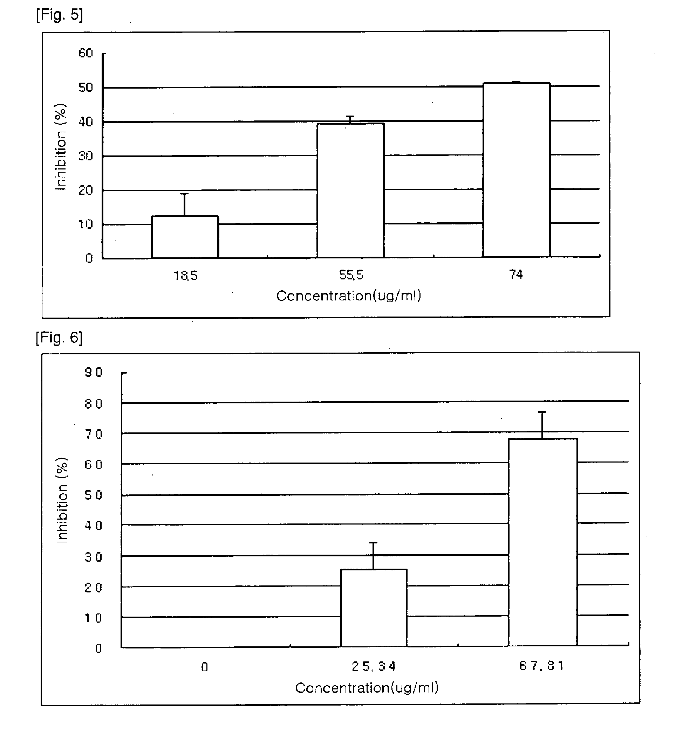

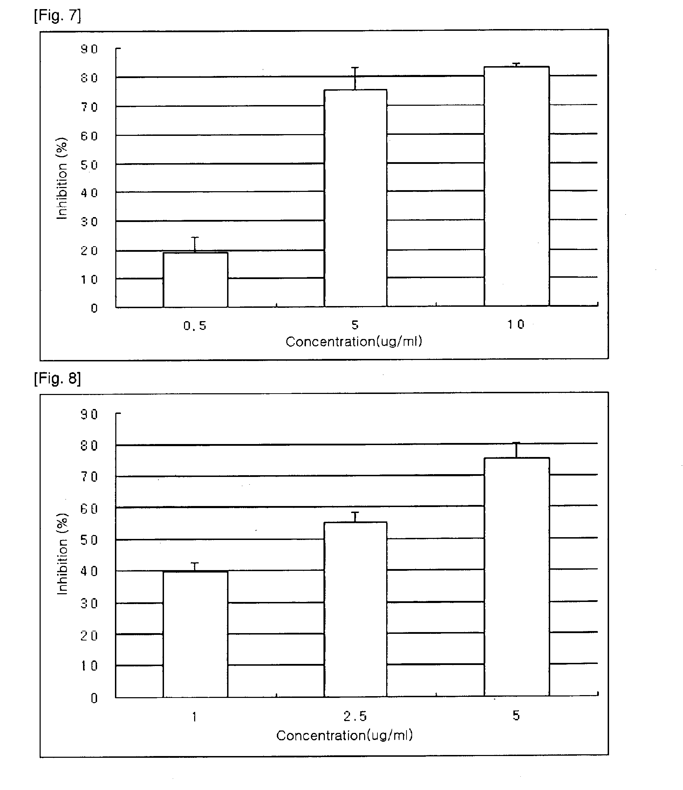

As shown in Table 2 and FIGS. 6 to 9, the leaf and stem extract of *Aster koraiensis* showed an $IC_{50}$ value cf 0.40 µg/mℓ, and thus the inhibitory effect thereof was about 10-fold superior to the positive control group, 3,3-tetramethyleneglutaric acid ($IC_{50}$ of 4.50 µg/mℓ). Also, the inhibitory effect cf the flower extract ($IC_{50}$ cf 38.75 µg/mℓ) was at least 2-fold superior to 3,3-tetramethyleneglutaric acid, and the inhibitory effect of the root extract ($IC_{50}$ of 3.77 µg/mℓ) was superior to 3,3-tetramethyleneglutaric acid.

2. Aldose reductase activity-inhibitory effect cf fractionated layers

The hexane fraction cf the root extract of *Aster koraiensis,* prepared in the above Example, was prepared into solutions having concentrations of 5.0 µg/mℓ, 10.0 µg/mℓ and 20.0 µg/mℓ. Then, each of the solutions was tested according to the method of Test Example 2 to measure the inhibitory effect thereof.

The ethyl acetate fraction of the root extract of *Aster koraiensis,* prepared in the above Example, was prepared into solutions having concentrations of 0.25 µg/mℓ, 0.5 µg/mℓ and 1.0 µg/mℓ. Then, each of the solutions was tested according to the method of Test Example 2 to measure the inhibitory effect thereof.

The n-butanol fraction of the root extract cf *Aster koraiensis,* prepared in the above Example, was prepared into solutions having concentrations of 0.25 µg/mℓ, 0.5 µg/mℓ and 1.0 µg/mℓ. Then, each of the solutions was tested according to the method of Test Example 2 to measure the inhibitory effect thereof.

The water fraction of the root extract cf *Aster koraiensis,* prepared in the above Example, was prepared into solutions having concentrations of 20.0 µg/mℓ, 30.0 µg/mℓ and 40.0 µg/mℓ. Then, each of the solutions was tested according to the method of Test Example 2 to measure the inhibitory effect thereof.

The hexane fraction of the flower extract of *Aster koraiensis,* prepared in the above Example, was prepared into solutions having concentrations of 10.0 $\mu$g/m$\ell$, 20.0 $\mu$g/m$\ell$ and 30.0 $\mu$g/m$\ell$. Then, each of the solutions was tested according to the method of Test Example 2 to measure the inhibitory effect thereof.

The ethyl acetate fraction of the flower extract cf *Aster koraiensis,* prepared in the above Example, was prepared into solutions having concentrations of 0.5 $\mu$g/m$\ell$, 1.0 $\mu$g/m$\ell$ and 2.5 $\mu$g/m$\ell$. Then, each of the solutions was tested according to the method of Test Example 2 to measure the inhibitory effect thereof.

The n-butanol fraction of the flower extract cf *Aster koraiensis,* prepared in the above Example, was prepared into solutions having concentrations of 2.5 $\mu$g/m$\ell$, 5.0 $\mu$g/m$\ell$ and 10.0 $\mu$g/m$\ell$. Then, each of the solutions was tested according to the method of Test Example 2 to measure the inhibitory effect thereof.

The water fraction cf the flower extract cf *Aster koraiensis,* prepared in the above Example, was prepared into solutions having concentrations of 10.0 $\mu$g/m$\ell$, 20.0 $\mu$g/m$\ell$ and 30.0 $\mu$g/m$\ell$. Then, each of the solutions was tested according to the method of Test Example 2 to measure the inhibitory effect thereof.

Table 3

[Table 3]

| Samples | Inhibition (%) of production of advanced glycation endproducts | | IC50 inhibitory concentration ($\mu$g/ml) | See |
|---|---|---|---|---|
| | Concentration | Inhibition | | |
| Hexane fraction of leaf and stem extract of *Aster koraiensis* | 2.5 | 25.42 $\pm$ 1.47 | 7.33 | |
| | 5.0 | 39.38 $\pm$ 7.45 | | |
| | 10.0 | 62.71 $\pm$ 5.14 | | |
| Ethyl acetate fraction of leaf and stem extract of *Aster koraiensis* | 0.5 | 31.86 $\pm$ 7.55 | 1.34 | FIG.10 |
| | 1.0 | 46.02 $\pm$ 11.29 | | |
| | 2.5 | 71.68 $\pm$ 4.27 | | |
| n-butanol fraction of leaf and stem extract of *Aster koraiensis* | 0.25 | 43.13 $\pm$ 5.29 | 0.47 | FIG.11 |
| | 0.5 | 52.29 $\pm$ 1.75 | | |
| | 1.0 | 62.98 $\pm$ 3.50 | | |
| Water fraction of leaf and stem extract of *Aster koraiensis* | 1.0 | 41.62 $\pm$ 7.20 | 2.15 | FIG.12 |
| | 2.5 | 54.82 $\pm$ 3.43 | | |
| | 5.0 | 65.23 $\pm$ 5.18 | | |
| Hexane fraction of root extract of *Aster koraiensis* | 5.0 | 22.86 $\pm$ 8.57 | 14.16 | |
| | 10.0 | 42.86 $\pm$ 6.97 | | |
| | 20.0 | 64.29 $\pm$ 2.70 | | |
| Ethyl acetate fraction of root extract of *Aster koraiensis* | 0.25 | 44.82 $\pm$ 2.70 | 0.41 | FIG.13 |
| | 0.5 | 52.66 $\pm$ 7.63 | | |
| | 1.0 | 71.5 $\pm$ 9.85 | | |
| n-butanol fraction of root extract of *Aster koraiensis* | 0.25 | 29.71 $\pm$ 5.39 | 0.86 | FIG. 14 |
| | 0.5 | 33.82 $\pm$ 4.91 | | |
| | 1.0 | 55.88 $\pm$ 4.91 | | |
| Water fraction of root extract of *Aster koraiensis* | 20.0 | 46.88 $\pm$ 3.84 | 20.23 | |
| | 30.0 | 75.00 $\pm$ 7.73 | | |
| | 40.0 | 87.22 $\pm$ 10.23 | | |
| Hexane fraction of flower extract of *Aster koraiensis* | 10.0 | 38.66 $\pm$ 3.83 | 14.02 | |
| | 20.0 | 66.45 $\pm$ 1.92 | | |
| | 30.0 | 99.36 $\pm$ 3.37 | | |

(continued)

| Samples | Inhibition (%) of production of advanced glycation endproducts | | IC50 inhibitory concentration ($\mu$g/ml) | See |
|---|---|---|---|---|
| | Concentration | Inhibition | | |
| Ethyl acetate fraction of flower extract of *Aster koraiensis* | 0.5 | 21.35 ± 8.63 | 1.39 | FIG.15 |
| | 1.0 | 43.42 ± 9.20 | | |
| | 2.5 | 80.43 ± 4.27 | | |
| n-butanol fraction of flower extract of *Aster koraiensis* | 2.5 | 48.44 ± 3.25 | 3.63 | FIG. 16 |
| | 5.0 | 51.25 ± 0.00 | | |
| | 10.0 | 72.19 ± 4.23 | | |
| Water fraction of flower extract of *Aster koraiensis* | 10.0 | 23.51 ± 1.71 | 22.40 | |
| | 20.0 | 46.64 ± 5.05 | | |
| | 30.0 | 64.93 ± 9.05 | | |

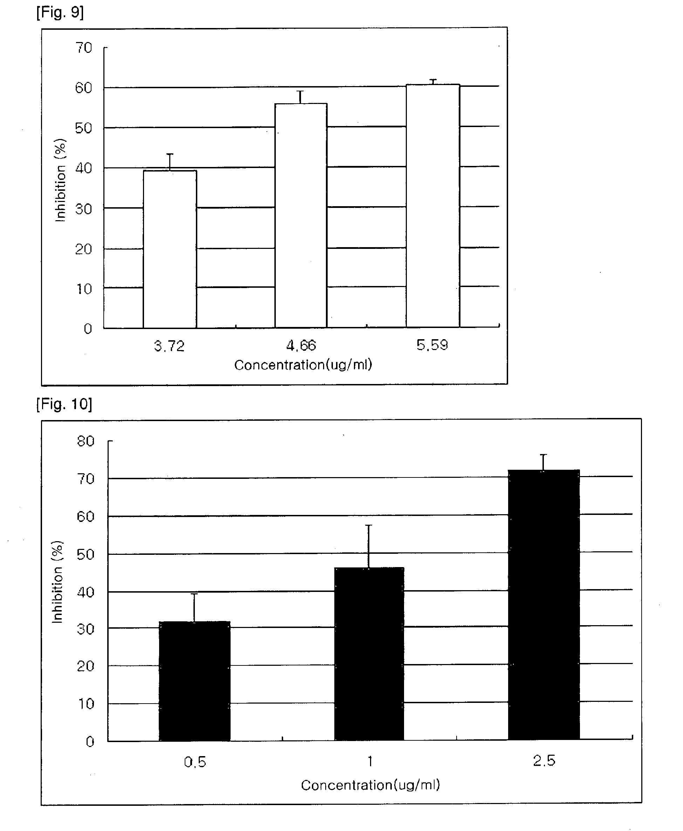

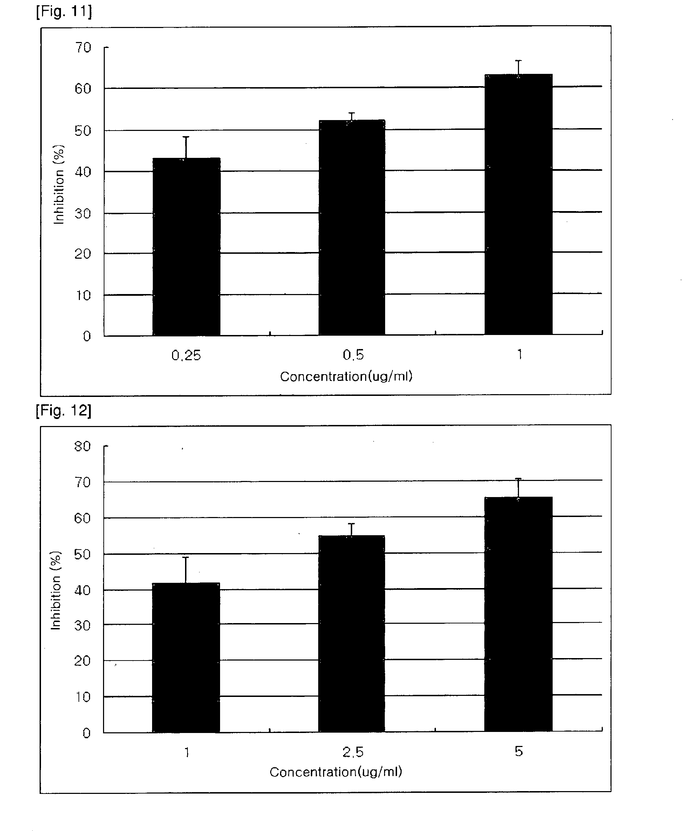

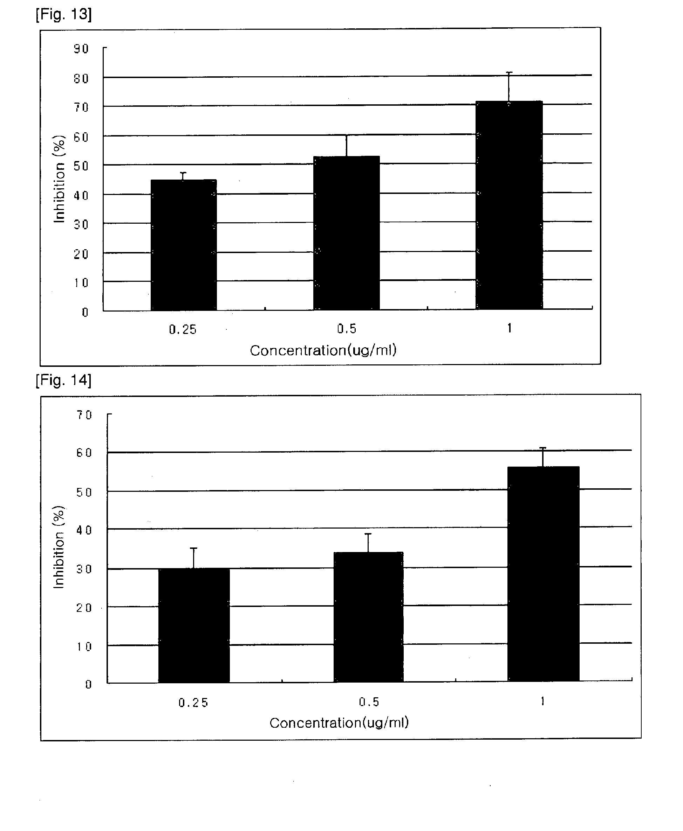

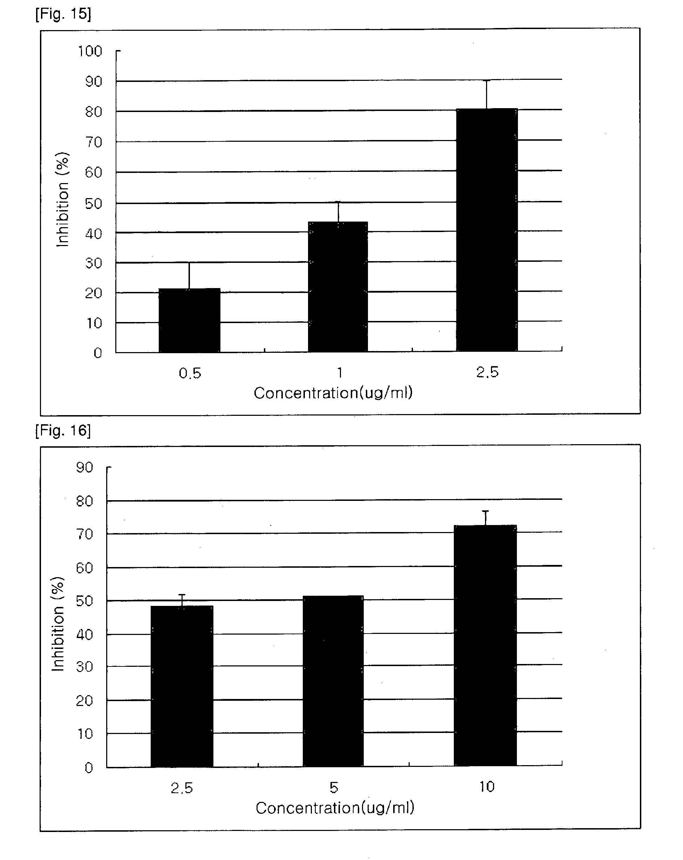

As shown in Table 3 and FIGS. 10 to 16, the $IC_{50}$ values of the n-butanol fraction, ethyl acetate fraction and water fraction cf the leaf and stem extract of *Aster koraiensis* were 0.47 $\mu$g/m$\ell$, 1.34 $\mu$g/m$\ell$ and 2.15 $\mu$g/m$\ell$, respectively, suggesting that the inhibitory effects of the fractions were 9.6-fold, 3.4-fold and 2.1-fold superior to 3,3-tetramethyleneglutaric acid ($IC_{50}$ of 4.50 $\mu$g/m$\ell$ shown in Table 2).

The $IC_{50}$ values of the ethyl acetate fraction and n-butanol fraction cf the root extract were 0.41 $\mu$g/m$\ell$ and 0.86 $\mu$g/m$\ell$, respectively, suggesting that the inhibitory effects cf the fractions were 11-fold and 5.2-fold superior to 3,3-tetramethyleneglutaric acid.

The $IC_{50}$ values cf the ethyl acetate fraction and n-butanol fraction cf the flower extract were 1.39 $\mu$g/m$\ell$ and 3.63 $\mu$g/m$\ell$, respectively, suggesting that the inhibitory effects of the fractions were 3.2-fold and 1.2-fold superior to 3,3-tetramethyleneglutaric acid.

Test Example 3: Inhibitory effect of inventive extracts and fractions on production of AGEs-BSA, measured using ELISA essay

Among the extracts and fractions according to the present invention, materials having a relatively excellent inhibitory effect on the production cf advanced glycation endproducts were analyzed for their effects according to the immunological method (ELISA) using antibodies specific for advanced glycation endproducts.

[Test method]

AGEs-ELISA was carried out according to the Engvall s method in the following manner.

100 $\mu\ell$ cf a mixture of a sample with 50 mM carbonate buffer (pH 9.6) (1:2 v/v) was added to each well of a 96-well plate, and then allowed to stand at 37 °C for 2 hours. The plate was washed three times with 0.05 % PBST, and then blocked with 1 % BSA/ PBS. After the plate was allowed to stand for 1 hour, it was washed three times with 0.05 % PBST. Each cf an AGE antibody (6D12, TransGenic Inc., Kobe, Japan) and a CML antibody (NF-1G, TransGenic Inc., Kobe, Japan) was 1000-fold diluted, and then 100 $\mu\ell$ of each cf the antibody dilutions was loaded in each well and allowed to stand at room temperature for 1 hour. After the plate was washed three times with 0.05 % PBST, a HRP-conjugate secondary antibody (Santa Cruz, USA) was 1000-fold diluted, and 100 $\mu\ell$ cf the antibody dilution was loaded in each well and allowed to stand for 1 hour. After the plate was washed three times with 0.05 % PBST, 100 $\mu\ell$ cf a substrate, 3,3', 5,5'-tetramethylbenzidine (TMB) was added to each well. After the plate was allowed to stand for 5 minutes, 100 $\mu\ell$ cf a reaction stopper solution (1M $H_2SO_2$) was added to each well. Then, the content in the plate was measured for absorbance using an ELISA reader.

[Test results]

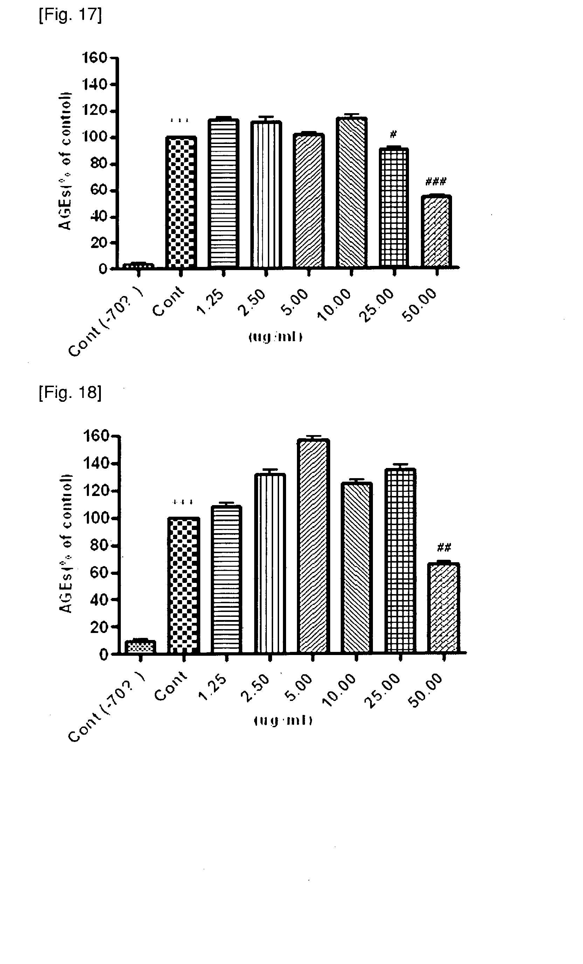

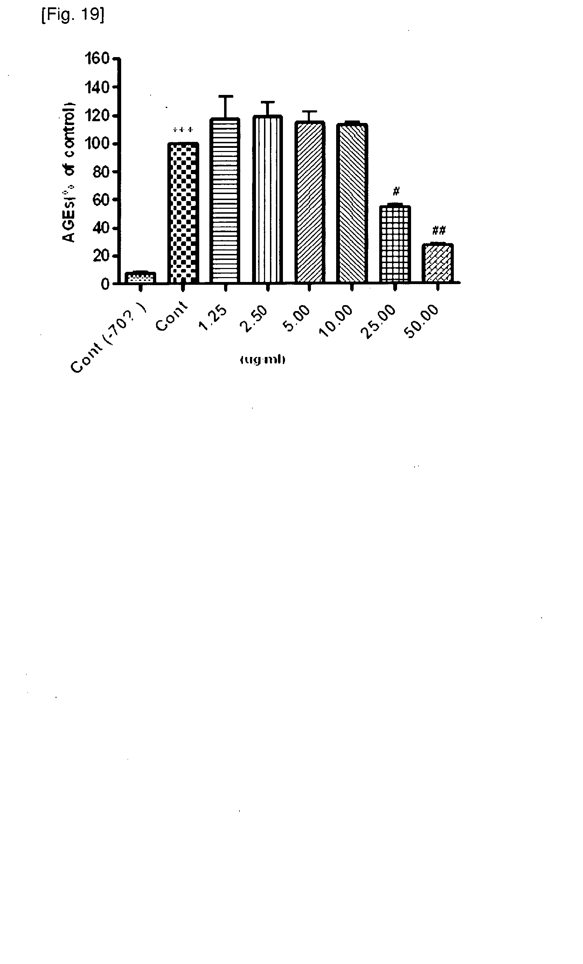

In the case of the root extract and the stem/leaf extract, cultured for 14 days, they showed significant inhibitory effects on the production cf AGEs at concentrations of 25 and 50 $\mu$g/m$\ell$ (see FIGS. 17 and 18). In the case of the flower extract, it showed a significant inhibitory effect on the production of AGEs at a concentrations of 50 $\mu$g/m$\ell$ (see FIG. 19).

In view cf the contents cf AGEs at the concentrations of 25 and 50 $\mu$g/m$\ell$, the stem/ leaf extract showed the most excellent effect.

In the figures, #, ## and ### indicate the statistical comparison of $p<0.05,$ 0.01 and 0.001 vs. culture containing no *Aster koraiensis.*

## Claims

1.  An extract of *Aster koraiensis* for use in the treatment or the prevention of diabetic complications.

2.  The extract of *Aster koraiensis* for use according to claim 1 wherein the extract of *Aster koraiensis* is any one selected from the group consisting of a leaf and stem extract of *Aster koraiensis,* a root extract of *Aster koraiensis,* a flower extract of *Aster koraiensis,* an ethyl acetate fraction, n-butanol fraction or water fraction of the leaf and stem extract of *Aster koraiensis,* an ethyl acetate fraction or n-butanol fraction of the root extract of *Aster koraiensis,* and an ethyl acetate fraction or n-butanol extract of the flower extract of *Aster koraiensis,* in which each of the extracts is obtained by finely cutting the leaf and stem part, root or flower of *Aster koraiensis,* extracting the cut plant with alcohol or aqueous alcohol solution, filtering the extracted solution and concentrating the filtrate under reduced pressure, and the fractions of each of the extracts are obtained by fractionating each of the extracts in the order of hexane, ethyl acetate, n-butanol and water fractions.

3.  The extract for use according to claim 1 or 2 wherein the diabetic complications is any one selected from the group consisting of diabetic retinopathy, diabetic cataract, diabetic nephropathy and diabetic neuropathy.

4.  A pharmaceutical composition for use in the prevention or the treatment of diabetic complications, which contains, as an active ingredient, an extract of *Aster koraiensis* according to claim 2.

5.  The pharmaceutical composition for use according to claim 1, wherein the diabetic complications is any one selected from the group consisting of diabetic retinopathy, diabetic cataract, diabetic nephropathy and diabetic neuropathy.

6.  A health functional food for use in the prevention and the alleviation of diabetic complications, which contains, as an active ingredient, an extract of *Aster koraiensis* according to claim 2.

7.  Non-therapeutic use of an extract of *Aster koraiensis* for the prevention or the delay of ageing.

8.  Non-therapeutic use of claim 7, wherein the extract of *Aster koraiensis* is any one selected from the group consisting of a leaf and stem extract of *Aster koraiensis,* a root extract of *Aster koraiensis,* a flower extract of *Aster koraiensis,* an ethyl acetate fraction, n- butanol fraction or water fraction of the leaf and stem extract of *Aster koraiensis,* an ethyl acetate fraction or n-butanol fraction of the root extract of *Aster koraiensis,* and an ethyl acetate fraction or n-butanol extract of the flower extract of *Aster koraiensis,* in which each of the extracts is obtained by finely cutting the leaf and stem part, root or flower of *Aster koraiensis,* extracting the cut plant with alcohol or aqueous alcohol solution, filtering the extracted solution and concentrating the filtrate under reduced pressure, and the fractions of each of the extracts are obtained by fractionating each of the extracts in the order of hexane, ethyl acetate, n-butanol and water fractions.

9.  Non-therapeutic use of a health functional food for the prevention or the delay of ageing, which contains, as an active ingredient, an extract of *Aster koraiensis* according to claim 8.

## Patentansprüche

1.  Extrakt von *Aster koraiensis* zur Verwendung bei der Behandlung oder der Verhütung von diabetesbedingten Komplikationen.

2.  Extrakt von *Aster koraiensis* zur Verwendung nach Anspruch 1, wobei es sich bei dem Extrakt von *Aster koraiensis* um einen beliebigen handelt, der aus der Gruppe ausgewählt ist, welche aus einem Blätter- und Stängelextrakt von *Aster koraiensis,* einem Wurzelextrakt von *Aster koraiensis,* einem Blütenextrakt von *Aster koraiensis,* einer Ethylacetatfraktion, n-Butanolfraktion oder Wasserfraktion des Blätter- und Stängelextrakts von *Aster koraiensis,* einer Ethylacetatfraktion oder n-Butanolfraktion des Wurzelextrakts von *Aster koraiensis* und einer Ethylacetatfraktion oder n-Butanolfraktion des Blütenextrakts von *Aster koraiensis* besteht, wobei die Extrakte jeweils erhalten werden, indem die Blätter- und Stängelteile, die Wurzel oder die Blüte von *Aster koraiensis* fein zerschnitten, die zerschnittene Pflanze mit Alkohol oder einer wässrigen Alkohollösung extrahiert, die extrahierte Lösung filtriert und das Filtrat unter vermindertem Druck aufkonzentriert wird, und wobei die Fraktionen der jeweiligen Extrakte erhalten werden, indem die Extrakte jeweils derart fraktioniert werden, dass der Reihe nach Hexan-, Ethylacetat-, n-Butanol- und Wasserfraktionen erhalten werden.

3. Extrakt zur Verwendung nach Anspruch 1 oder 2, wobei es sich bei den diabetesbedingten Komplikationen um eine beliebige handelt, die aus der Gruppe ausgewählt ist, welche aus diabetesbedingter Retinopathie, diabetesbedingtem Katarakt, diabetesbedingtem Nierenleiden und diabetesbedingter Neuropathie besteht.

4. Pharmazeutische Zusammensetzung zur Verwendung bei der Verhütung oder der Behandlung diabetesbedingter Komplikationen, wobei diese als Wirkbestandteil einen Extrakt von *Aster koraiensis* nach Anspruch 2 enthält.

5. Pharmazeutische Zusammensetzung zur Verwendung nach Anspruch 1, wobei es sich bei den diabetesbedingten Komplikationen um eine beliebige handelt, die aus der Gruppe ausgewählt ist, welche aus diabetesbedingter Retinopathie, diabetesbedingtem Katarakt, diabetesbedingtem Nierenleiden und diabetesbedingter Neuropathie besteht.

6. Funktionelles Lebensmittel mit Gesundheitswirkung zur Verwendung bei der Verhütung oder der Linderung diabetesbedingter Komplikationen, wobei dieses als Wirkbestandteil einen Extrakt von *Aster koraiensis* nach Anspruch 2 enthält.

7. Nicht-therapeutische Verwendung eines Extraktes von *Aster koraiensis* zur Verhütung oder Verzögerung des Alterns.

8. Nicht-therapeutische Verwendung nach Anspruch 7, wobei es sich bei dem Extrakt von *Aster koraiensis* um einen beliebigen handelt, der aus der Gruppe ausgewählt ist, welche aus einem Blätter- und Stängelextrakt von *Aster koraiensis,* einem Wurzelextrakt von *Aster koraiensis,* einem Blütenextrakt von *Aster koraiensis,* einer Ethylacetatfraktion, n-Butanolfraktion oder Wasserfraktion des Blätter- und Stängelextrakts von *Aster koraiensis,* einer Ethylacetatfraktion oder n-Butanolfraktion des Wurzelextrakts von *Aster koraiensis* und einer Ethylacetatfraktion oder n-Butanolfraktion des Blütenextrakts von *Aster koraiensis* besteht, wobei die Extrakte jeweils erhalten werden, indem die Blätter- und Stängelteile, die Wurzel oder die Blüte von *Aster koraiensis* fein zerschnitten, die zerschnittene Pflanze mit Alkohol oder einer wässrigen Alkohollösung extrahiert, die extrahierte Lösung filtriert und das Filtrat unter vermindertem Druck aufkonzentriert wird, und wobei die Fraktionen der jeweiligen Extrakte erhalten werden, indem die Extrakte jeweils derart fraktioniert werden, dass der Reihe nach Hexan-, Ethylacetat-, n-Butanol- und Wasserfraktionen erhalten werden.

9. Nicht-therapeutische Verwendung eines funktionellen Lebensmittels mit Gesundheitswirkung zur Verhütung oder Verzögerung des Alterns, wobei dieses als Wirkbestandteil einen Extrakt von *Aster koraiensis* nach Anspruch 8 enthält.

**Revendications**

1. Extrait *d'Aster koraiensis* pour une utilisation dans le traitement ou la prévention de complications diabétiques.

2. Extrait *d'Aster koraiensis* pour une utilisation selon la revendication 1 dans lequel l'extrait d' *Aster koraiensis* est l'un quelconque choisi dans le groupe constitué par un extrait de feuille et de tige *d'Aster koraiensis,* un extrait de racine *d'Aster koraiensis,* un extrait de fleur *d'Aster koraiensis,* une fraction d'acétate d'éthyle, une fraction de n-butanol ou une fraction aqueuse de l'extrait de feuille et de tige *d'Aster koraiensis,* une fraction d'acétate d'éthyle ou une fraction de n-butanol de l'extrait de racine d'*Aster koraiensis* et une fraction d'acétate d'éthyle ou un extrait de n-butanol de l'extrait de fleur d'*Aster koraiensis,* dans lesquels chacun des extraits est obtenu en découpant finement la partie de feuille et de tige, la racine ou la fleur *d'Aster koraiensis,* en extrayant la plante découpée avec de l'alcool ou une solution alcoolique aqueuse, en filtrant la solution extraite et en concentrant le filtrat sous pression réduite, et les fractions de chacun des extraits sont obtenues en fractionnant chacun des extraits dans l'ordre de l'hexane, de l'acétate d'éthyle, du n-butanol et des fractions aqueuses.

**3.** Extrait pour une utilisation selon la revendication 1 ou 2 dans lequel les complications diabétiques sont l'une quelconque choisie dans le groupe constitué par la rétinopathie diabétique, la cataracte diabétique, la néphropathie diabétique et la neuropathie diabétique.

**4.** Composition pharmaceutique pour une utilisation dans la prévention ou le traitement de complications diabétiques, qui contient, comme principe actif, un extrait *d'Aster koraiensis* selon la revendication 2.

**5.** Composition pharmaceutique pour une utilisation selon la revendication 1, dans laquelle les complications diabétiques sont l'une quelconque choisie dans le groupe constitué par la rétinopathie diabétique, la cataracte diabétique, la néphropathie diabétique et la neuropathie diabétique.

**6.** Aliment fonctionnel de santé pour une utilisation dans la prévention et le soulagement de complications diabétiques, qui contient, comme principe actif, un extrait *d'Aster koraiensis* selon la revendication 2.

**7.** Utilisation non thérapeutique d'un extrait *d'Aster koraiensis* pour la prévention ou le retardement du vieillissement.

**8.** Utilisation non thérapeutique selon la revendication 7, dans laquelle l'extrait d'Aster koraiensis est l'un quelconque choisi dans le groupe constitué par un extrait de feuille et de tige *d'Aster koraiensis,* un extrait de racine d'*Aster koraiensis,* un extrait de fleur *d'Aster koraiensis,* une fraction d'acétate d'éthyle, une fraction de n-butanol ou une fraction aqueuse de l'extrait de feuille et de tige *d'Aster koraiensis,* une fraction d'acétate d'éthyle ou une fraction de n-butanol de l'extrait de racine *d'Aster koraiensis* et une fraction d'acétate d'éthyle ou un extrait de n-butanol de l'extrait de fleur *d'Aster koraiensis,* dans lesquels chacun des extraits est obtenu en découpant finement la partie de feuille et de tige, la racine ou la fleur *d'Aster koraiensis,* en extrayant la plante découpée avec de l'alcool ou une solution alcoolique aqueuse, en filtrant la solution extraite et en concentrant le filtrat sous pression réduite, et les fractions de chacun des extraits sont obtenues en fractionnant chacun des extraits dans l'ordre de l'hexane, de l'acétate d'éthyle, du n-butanol et des fractions aqueuses.

**9.** Utilisation non thérapeutique d'un aliment fonctionnel de santé pour la prévention ou le retardement du vieillissement, qui contient, comme principe actif, un extrait *d'Aster koraiensis* selon la revendication 8.

[Fig. 1]

[Fig. 2]

**EP 2 131 850 B1**

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

[Fig. 17]

[Fig. 18]

[Fig. 19]

## REFERENCES CITED IN THE DESCRIPTION

**Non-patent literature cited in the description**

- **VINSON, J. A. et al.** *J. Nutritinal Biochemistry,* 1996, vol. 7, 559-663
- **SMITH, P. R. et al.** *Eur. J. Biochem.,* 1992, vol. 210, 729-739
- **BROWNLEE, M. et al.** *Sciences,* 1986, vol. 232, 1629-1632
- **YOKOZAWA, T. et al.** *J. of Trad. Med.,* 2001, vol. 18, 107-112
- **EUNGJN KIM et al.** diabetics. Korean diabetes association, 483
- **SOULIS-LIPAROTA, T. et al.** *Di- abetologia,* 1995, vol. 38, 357-394
- **NAKAMURA, N. et al.** *Free Radic Biol. Med.,* 2000, vol. 29, 17-25
- **BROWNLEE, M. et al.** *N. Engl. Med,* 1988, vol. 318, 1315-1321
- **EDELSTEIN, D. et al.** *Diabetes,* 1992, vol. 41, 26-29
- **PARK HEE-JUHN.** Chemistry and Pharmacological Action of Caffeoylquinic Acid Derivatives and Pharmaceutical Utilization of Chwinamul (Korean Mountainous Vegetable). *Archives Of Pharmacol. Research (Seoul),* November 2010, vol. 33 (11), 1703-1720
- **SEI-YOUNG, YUN.** Pictorial Book of Korean Plants. Academic Press, 508